(19) Europäisches Patentamt
European Patent Office
Office européen des brevets

(11) Numéro de publication : **0 194 906**
**B1**

(12) **FASCICULE DE BREVET EUROPÉEN**

(45) Date de publication du fascicule du brevet : **19.07.89**

(21) Numéro de dépôt : **86400273.8**

(22) Date de dépôt : **10.02.86**

(51) Int. Cl.[4] : **C 07 D405/12**, C 07 D411/12, C 07 D409/12, C 07 D327/04, C 07 D317/24

(54) **Esters hérérocycliques dissymétriques d'acides 1,4-dihydropyridine-3,5-dicarboxyliques, procédé de préparation et utilisation en thérapeutique.**

(30) Priorité : **20.02.85 FR 8502412**

(43) Date de publication de la demande : **17.09.86 Bulletin 86/38**

(45) Mention de la délivrance du brevet : **19.07.89 Bulletin 89/29**

(84) Etats contractants désignés : **AT BE CH DE FR GB IT LI LU NL SE**

(56) Documents cités :
**FR–A– 2 514 761**
**JOURNAL OF PHARMACEUTICAL SCIENCES, vol. 72, no. 3, mars 1983, pages 280-285, American Pharmaceutical Association; TOSHIAKI NISHIHATA et al.: "Adjuvant effects of glyceryl esters of acetoacetic acid on rectal absorption of insulin and inulin in rabbits"**

(73) Titulaire : **FOURNIER INNOVATION ET SYNERGIE**
**38, avenue Hoche**
**F-75008 Paris (FR)**

(72) Inventeur : **Robin, Jacques**
**19 rue de la Corvée**
**F-21000 Dijon (FR)**
Inventeur : **Pruneau, Didier**
**31 rue Docteur Lavalle**
**F-21000 Dijon (FR)**
Inventeur : **Bonenfant, Alain**
**2 rue Edouard Rod**
**CH-1203 Génève (CH)**

(74) Mandataire : **Clisci, Serge et al**
**S.A. FEDIT-LORIOT CONSEILS EN PROPRIETE INDUSTRIELLE 38, avenue Hoche**
**F-75008 Paris (FR)**

**EP 0 194 906 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a trait à de nouveaux dérivés appartenant à la famille des esters hétérocycliques dissymétriques d'acides 1,4-dihydropyridine-3,5-dicarboxyliques. Elle concerne également l'utilisation en thérapeutique et le procédé de préparation de ces nouveaux dérivés.

On sait que la nifédipine, qui répond à la nomenclature systématique de 1,4-dihydro-2,6-diméthyl-4-(2-nitrophényl)-pyridine-3,5-dicarboxylate de diméthyle et est décrite dans US-A-3 485 847, est un agent vasodilatateur coronarien de référence. On sait également que la nicardipine, qui répond à la nomenclature systématique de 1,4-dihydro-2,6-diméthyl-4-(3-nitrophényl)-pyridine-3,5-dicarboxylate de méthyle et de 2-(N-méthyl-N-benzylamino)-éthyle et est décrite dans US-A- 985 758, est un vasodilatateur cérébral de référence. On sait enfin que l'on a préconisé dans FR-A-2 514 761 des esters dissymétriques d'acide 1,4-dihydro-2,6-diméthyl-pyridine-3,5-dicarboxylique de formule

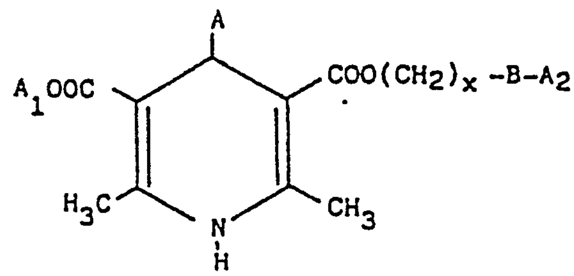

($I_0$)

où notamment A est 2- ou 3-nitrophényle ; $A_1$ est alkyle en $C_1$-$C_4$ ; $A_2$ est H ou $CH_3$ ; B est CO ou un groupe acétal ou thioacétal cyclique tel que éthylènedioxy, propylènedioxy, éthylènedithio ou propylènedithio, x est 1 ou 2, en tant qu'agents vasodilatateurs coronariens et cérébraux.

On vient de trouver à présent que les dérivés esters dissymétriques selon l'invention sont des agents vasodilatateurs qui diffèrent du dérivé symétrique antérieurement connu, la nifédipine, et des dérivés dissymétriques, la nicardipine et les acétals et thioacétals cycliques de formule $I_0$ précités, par (i) leur structure, et (ii) leurs propriétés thérapeutiques bénéfiques en ce qui concerne notamment leurs effets antihypertenseurs et leurs effets sur les débits cardiaque, fémoral et coronaire.

Les nouveaux dérivés selon l'invention sont caractérisés en ce qu'ils sont choisis parmi l'ensemble constitué par

(i) les dérivés esters hétérocycliques dissymétriques d'acide 1,4-dihydro-2,6-diméthyl-pyridine-3,5-dicarboxylique de formule générale

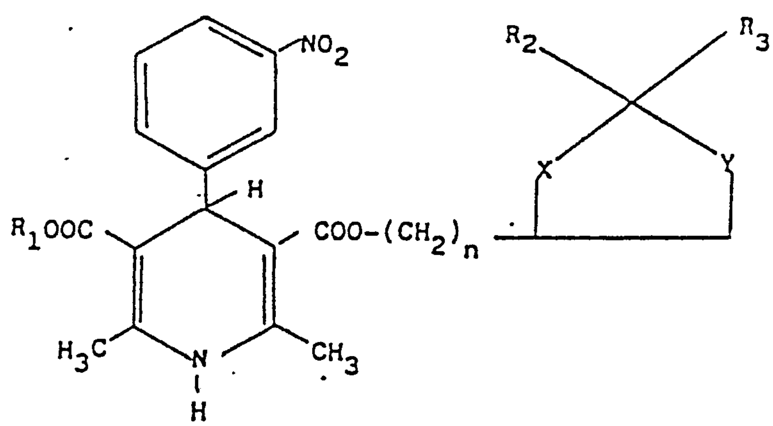

(I)

dans laquelle

$R_1$ est un groupe alkyle en $C_1$-$C_4$ ;

X et Y identiques ou différents représentent chacun O ou S, un au moins des X et Y étant différent de S ;

n est un nombre entier valant 1 ou 2 ;

$R_2$ et $R_3$, identiques ou différents, représentent chacun l'atome d'hydrogène, le groupe méthyle, le groupe trifluorométhyle, le groupe phényle ou un groupe halogénophényle, $R_2$ et $R_3$ considérés ensemble pouvant former avec l'atome de carbone de l'hétérocycle auquel ils sont liés un groupe spirocycloaliphatique de 5 à 7 sommets ;

(ii) leurs isomères optiques et diastéréoisomères.

Parmi les groupes cycloaliphatiques inclus dans la définition des groupes $R_2$ et $R_3$ considérés ensemble, on peut notamment mentionner les groupes cyclopentyle, cyclohexyle et cycloheptyle.

Parmi les atomes d'halogène du groupe halogénophényle inclus dans la définition des groupes $R_2$ et $R_3$, on peut citer les atomes de fluor, de chlore et de brome. Parmi les groupes halogénophényle qui conviennent, on peut mentionner notamment les groupes 4-chlorophényle, 3-fluorophényle, 2,4-dichlorophényle et 2,4-dibromophényle.

Les composés de formule I ci-dessus peuvent être préparés selon une méthode connue en soi par application de mécanismes réactionnels classiques. Selon l'invention on préconise un procédé qui consiste à faire réagir un nitrobenzylidène-acétoacétate de formule

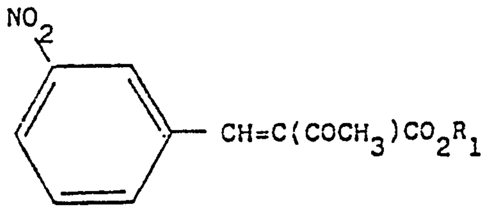

(II)

où $R_1$ est défini comme indiqué ci-dessus, avec un aminocrotonate de formule

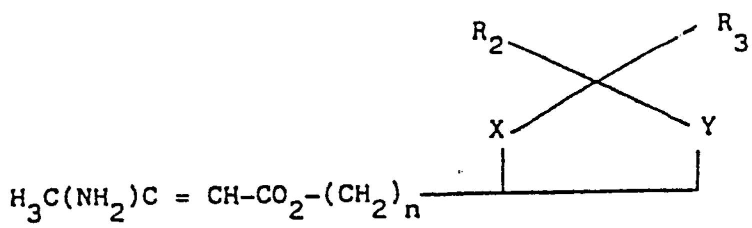

(III)

où n, X, Y, $R_2$ et $R_3$ sont définis comme indiqué ci-dessus, selon la réaction dite de HANTZSCH.

De façon avantageuse on fait réagir environ 1 mole de II avec environ 1 mole de III dans un solvant organique polaire (notamment un alkanol en $C_1$-$C_4$ tel que méthanol, éthanol, isopropanol, n-butanol, t-butanol), pendant 1 à 24 h, à une température comprise entre la température ambiante (15-20°C) et la température de reflux du milieu réactionnel.

Les composés de formule II sont des substances connues qui peuvent être préparées selon la réaction dite de Knoevenagel, voir à cet effet l'artile récapitulatif de G. Jones, Organic Reactions 15, 204 (1975).

Les composés de formule III sont des produits nouveaux que l'on préconise de préparer comme suit

1) réaction d'un alcool de formule

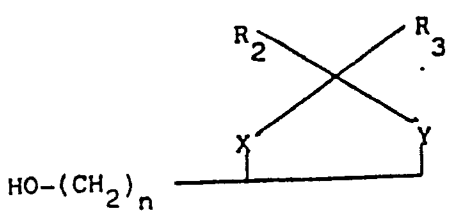

(IV)

où n, X, Y, $R_2$ et $R_3$ sont définis comme indiqué ci-dessus, avec le 5-acétyl-2,2-diméthyl-4,6-dioxo-1,3-dioxanne de formule

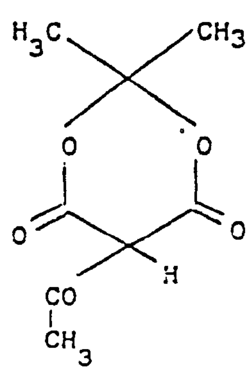

(V)

dans un solvant aromatique (notamment le benzène, le toluène, les xylènes) à une température comprise entre la température ambiante (15-20 °C) et la température de reflux du milieu réactionnel, pendant 1 à 24 h, pour obtenir un céto-ester de formule

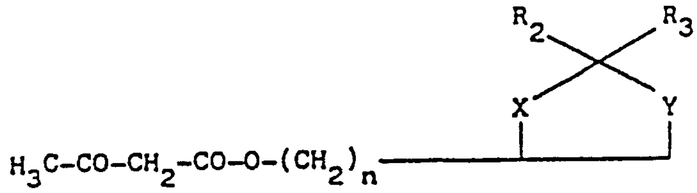

(VI)

et,

2) traitement du dérivé de formule VI ainsi obtenu au moyen de $NH_3$ dans un solvant chloré (notamment $CH_2Cl_2$, $CHCl_3$) pendant 1 à 20 h.

Les composés de formules III et IV peuvent être utilisés sans avoir été préalablement purifiés. De façon avantageuse on les utilise après les avoir purifiés soit par distillation (ou recristallisation) soit par « flash chromatography » (chromatographie sur colonne et sous pression) selon la technique décrite par W. C. Still et al., J. Org. Chem. 43 (N° 14), 2923 (1978).

Dans les tableaux I et II ci-après on a consigné de façon non limitative les céto-esters de formule VI et respectivement les aminocrotonates de formule III, qui ont été préparés comme indiqué ci-dessus et qui interviennent dans la synthèse des composés selon l'invention.

On connaît de l'article de Toshiaki Nishihata et al., J. Pharm. Sci., 72 (N° 3), 280-285, (1983), un composé de formule VI où $n = 1$, $R_2 = R_3 = CH_3$ et $X = Y = 0$, à savoir l'acétoacétate de (2,2-diméthyl-1,3-dioxolanne-4-yl) méthyle [autre nomenclature : acétoacétate de 3-(1,2-isopropylidèneglycéryle)], ledit article concernant notamment l'effet de ce composé sur l'absorption rectale d'insuline et d'inciline chez le lapin. Les autres composés de formule VI sont nouveaux.

(Voir Tableaux I, II, III pages 5 à 11)

Tableau I

$$H_3C-\overset{O}{\overset{\|}{C}}-CH_2-\overset{O}{\overset{\|}{C}}-OR$$

| R | Temps de réaction (heures) | Rendement % | Méthode de purification | Point d'ébullition (°C) | RMN |
|---|---|---|---|---|---|
| 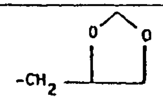 | 2 | 77 | distillation | 100-110(b) | - |
| 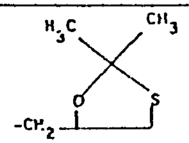 | 2 | 60 | "Flash chromatography" /¯hexane-acétone (75:5) v/v_7 | - | 1,6-1,66(6H,2 singulets);2,28 (3H,s);3,1(2H,m) 3,5(2H,s);4,4 (3H.m). |
| 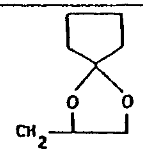 | 9 | 80 | (a) | - | 1,69(8H,m);2,27 (3H,s);3,5(2H,s); 3,6-4,3(5H,m) |
| 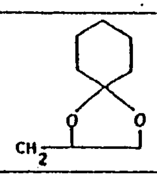 | 6 | 70 | "Flash chromatography" /¯hexane-acétone (95:5) v/v_7 | - | 1,48(10H,m);2,25 (3H,s);3,42(2H,s) 3,6-4,3(5H,m) |
| 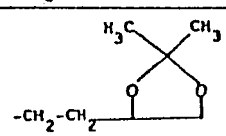 | 2 | 90 | (a) | - | 1,37(6H,2 singulets);1,96(2H,s) 2,26(3H,s);3,45 (2H,s);3,5-4,3 (5H,m). |
| 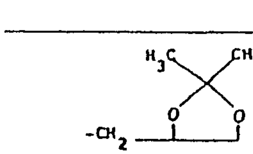 (d) | 15 | 85 | distillation | 95(c) | - |

Tableau I (Suite)

| R | Temps de réaction (heures) | Rendement % | Méthode de purification | Point d'ébullition | RMN |
|---|---|---|---|---|---|
| 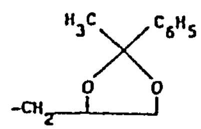 | 2 | 90 | (a) | - | - |
| 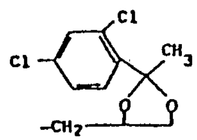 | 6 | 90 | (a) | - | - |
| 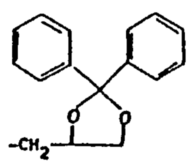 | 2 | 90 | (a) | - | - |
| 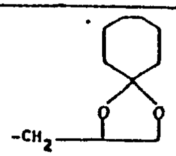 | 3 | 95 | (a) | - | - |
| 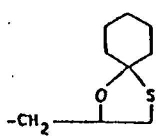 | 2 | 95 | (a) | - | - |

Tableau I (Suite)

| R | Temps de réaction (heures) | Rendement % | Méthode de purification | point d'ébullition | RMN |
|---|---|---|---|---|---|
| 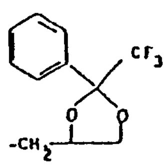 | 2 | 100(brut) | (a) | - | 2,14-2,28(3H,2singulets); 3,24-3,5 (2H,2 singulets); 3,8-4,8(5H, m) |
| 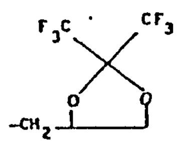 | 2 | 92(brut) | (a) | - | 2,26 (3H,s) ;3,5(2H,s); 3,9-4,6 (5H,m) |
| 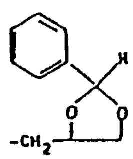 | 1 H 30 | 86(brut) | (a) | - | 2,20-2,25 (3H,d) 3,45-3,5 (d)- 3,6 (s)2H 3,6-4,5(5H,m);5,55-5,8-5,94(1H,3 singulets) ; 7,2-7,6(5H,m) |

Notes :
(a) sans purification préalable
(b) sous 2 mmHg (c'est-à-dire environ 26,6 Pa)
(c) sous 0.2 mmHg (c'est-à-dire environ 2,66 Pa)
(d) décrit par T. Nishihata et al., J. Pharm. Sci. 72 (N° 3), 280-285 (1983).

Tableau II

$H_3C(NH_2)C=CH-CO_2R$

| R | Temps de réaction (heures) | Rendement % | Methode de purification | RMN |
|---|---|---|---|---|
| 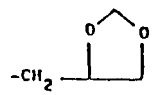 | 3 | 70 | (a) | 1,95(3H,s);3,7-4,3 (5H,m) ; 4,55(1H,s); 5(2H,2 singulets) |
| 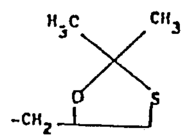 | 3 | 90 | (a) | 1,65(6H,s);1,9(3H,s); 2,9-3,15(2H,m);4,2-4,3(3H,m);4,55(1H,s) |
| 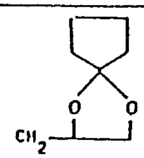 | 3 | 95 | (a) | 1,7(8H,m);1,9(3H,s); 3,5-4,2(5H,m);4,55 (1H,s) |
| 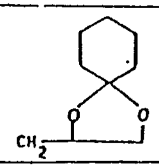 | 3 | 95 | (a) | 1,58(10H,m);1,9(3H,s) 3,7-4,2(5H,m);4,55 (1H,s) |
| 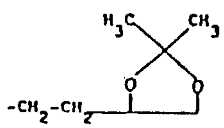 | 3 | 90 | "Flash Chromatography" / hexane-isopropanol(50:1) v/v / | 1,37(6H,2 singulets); 1,9(3H,s);1,95(2H,m); 3,56-4,3(5H,m);4,5 (1H,s) |
| 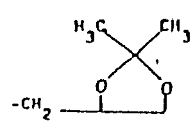 | 2 | 60 | recristallisation (b) | 1,37(6H,2 singulets); 1,9(3H,s); 3,7 -4,2(5H,m);4,56 (1H,s) |

Tableau II (Suite)

| R | Temps de réaction (heures) | Rendement % | Méthode de purification | RMN |
|---|---|---|---|---|
| 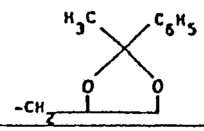 | 2 | 90 | (a) | - |
| 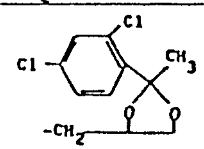 | 3 | 95 | (a) | - |
| 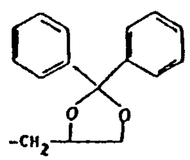 | 3 | 90 | (a) | - |
| 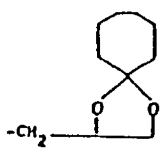 | 3 | 90 | (a) | - |
| 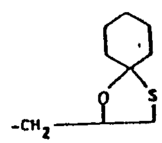 | 4 | 90 | (a) | - |

Tableau II (Suite)

| R | Temps de réaction (heures) | Rendement % | Méthode de purification | RMN |
|---|---|---|---|---|
| 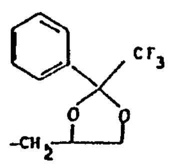 | 3 | 100 (brut) | (a) | 1,87-1,9 (3H, 2 singulets) 3,85 - 4,8 (6H, m) |
| 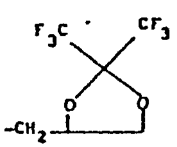 | 2 | 100 (brut) | (a) | 1,9 (3H,s) 3,9 - 4,7 (massif dont 1 singulet à 4,5 |
| 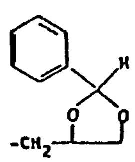 | 3 | 100 (brut) | (a) | 2,23-2,26 (3H,d) ;3,4-3,6(2H,m) 3,6 - 4,6 (1H,m) ; 5,55 -5,8-5,96 (1H,3 singulets); 7,2 - 7,6 (5H,m) |

Notes :
(a) sans purification
(b) point de fusion : 87 °C.

Tableau III

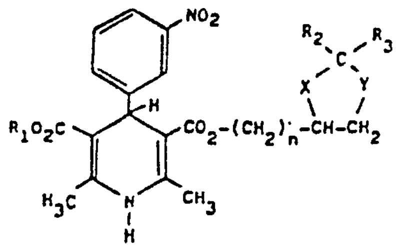

| Produit | Numéro de code | $R_1$ | $R_2$ | $R_3$ | n | X | Y | Point de fusion (°C) |
|---|---|---|---|---|---|---|---|---|
| Ex 1 | SR 1154 | $CH_3$ | $CH_3$ | $CH_3$ | 1 | O | O | 55 |
| Ex 2 | SR 1308 | $CH_3$ | $-(CH_2)_5-$ | | 1 | O | O | 62 |
| Ex 3 | SR 1344 | $CH_3$ | H | H | 1 | O | O | 119 |
| Ex 4 | SR 1377 | $CH_3$ | $CH_3$ | $CH_3$ | 2 | O | O | (a) |
| Ex 5 | SR 1399 | $CH_3$ | $-(CH_2)_4-$ | | 1 | O | O | (b) |
| Ex 6 | SR 1411 | $CH_3$ | $CH_3$ | $CH_3$ | 1 | O | S | 82 |
| Ex 7 | SR 1433 | $CH_3$ | $CH_3$ | 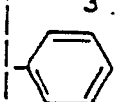 | 1 | O | O | 50 |
| Ex 8 | SR 1457 | $CH_3$ | 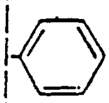 | 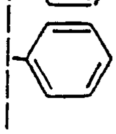 | 1 | O | O | 75 |
| Ex 9 | 2.0050 | $CH_3$ | $-(CH_2)_5-$ | | 1 | O | S | 50 |
| Ex 10 | 2.0054 | $CH_3$ | $-(CH_2)_6-$ | | 1 | O | O | 50 |
| Ex 11 | SR 1458 | $CH_3$ | $CH_3$ | 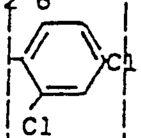 | 1 | O | O | 50 |
| Ex 12 | 2.0095 | $CH_3$ | $CF_3$ | 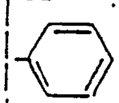 | 1 | O | O | (c) |
| Ex 13 | 2.0128 | $CH_3$ | $CF_3$ | $CF_3$ | 1 | O | O | 113 |
| Ex 14 | 2.0182 | $CH_3$ | H | 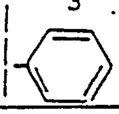 | 1 | O | O | (d) |

Notes :
(a) produit visqueux
(b) mousse de couleur jaune
(c) mousse fondant sur une plage de 65° à 80 °C
(d) mousse fondant sur une plage de 55° à 80 °C.

Les isomères énantiomères et diastéréoisomères sont séparés des racémiques correspondants selon une méthode connue en soi, notamment par cristallisation fractionnée, par dédoublement ou autre.

Selon l'invention on préconise une composition thérapeutique qui est caractérisée en ce qu'elle renferme, en association avec un excipient physiologiquement acceptable, au moins un composé choisi parmi l'ensemble constitué par les dérivés de formule I ci-dessus, les isomères énantiomères et les diastéréoisomères.

Bien entendu, dans une telle composition le principe actif intervient en quantité pharmaceutiquement efficace.

D'autres avantages et caractéristiques de l'invention seront mieux compris à la lecture qui va suivre d'exemples de préparation et de résultats d'essais pharmacologiques. L'ensemble de ces éléments n'est pas limitatif mais donné à titre d'illustration.

Préparation I

Obtention du 1,4-dihydro-2,6-diméthyl-4-(3-nitrophényl)-pyridine-3,5-dicarboxylate de méthyle et de (2,2-diméthyl-1,3-dioxolanne-4-yl)-méthyle.

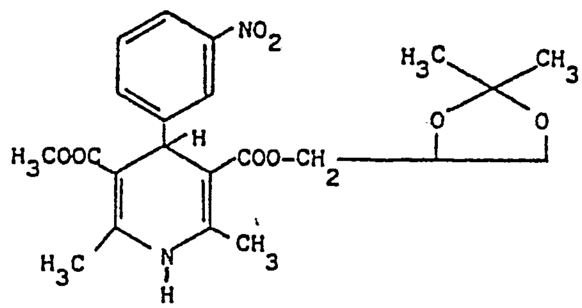

Exemple 1 ; N° de Code : SR 1154)

On chauffe à 95-100 °C pendant 13 heures un mélange de 2,5 g (0,01 mole) de 3-nitrobenzylidène-acétylacétate de méthyle, 2,2 g (0,01 mole) de 3-aminocrotonate de (2,2-diméthyl-1,3 dioxolanne-4-yl)-méthyle et 20 ml de tertiobutanol. Le solvant est évaporé et la mousse brute obtenue (3,4 g) est dissoute dans 5 ml d'acétone puis purifiée par chromatographie sur colonne de gel de silice selon la technique « flash chromatography » sus-visée en éluant avec un mélange acétone-hexane (1 : 5) v/v). On obtient 3,10 g (rendement : 69,5 %) de SR 1154. F = 55 °C.

Spectre de RMN ($CDCl_3$) δ (intensité, multiplicité) :

1,34 (3H,s) ; 1,39 (3H,s) ; 2,36 (6H,s) ; 3,6-4,2 (5H,m) ; 3,6 (3H,s) ; 5,12 (1H,s) ; 5,89 (1H,s) ; 7,4-8,09 (4H,m).

Préparation II

Obtention du 1,4-dihydro-2,6-diméthyl-4-(3-nitrophényl)-pyridine-3,5-dicarboxylate de méthyle et de (1,4-dioxa-spiro[4,5]décane-2-yl)méthyle (Exemple 2 ; N° de code : SR 1308)

On chauffe à 90-95 °C pendant 12 heures un mélange de 4,8 g (1,88 × $10^{-2}$ mole) de 3-nitrobenzylidène-acétylacétate de méthyle, 4,68 g (1,88 × $10^{-2}$ mole) de 3-aminocrotonate de (1,4-dioxa-spiro[4,5]décane-2-yl)méthyle et 40 ml de tertiobutanol. Le solvant est évaporé et le produit obtenu est purifié par « flash chromatography » en éluant avec un mélange acétone-hexane (1 : 5) v/v. On obtient 4,2 g (rendement : 46 %) de SR 1308. F = 62 °C.

Spectre de RMN ($CDCl_3$) δ (intensité, multiplicité) :

1,58 (10H,s) ; 2,37 (6H,s) ; 3,65-4,12 (5H,m) ; 3,65 (3H,s) ; 5,12 (1H,s) ; 5,80 (1H,s) ; 7,4-8,08 (4H,m).

Préparation III

Obtention du 1,4-dihydro-2,6-diméthyl-4-(3-nitrophényl)-pyridine-3,5-dicarboxylate de méthyle et de 2-[(2,2-diméthyl-1,3-dioxolanne-4-yl)-éthyle] (Exemple 4 ; N° de Code SR 1377).

On chauffe au reflux pendant 5 heures un mélange de 8 g (3,2 × $10^{-2}$ mole) de 3-nitrobenzylidène-acétylacétate de méthyle, 9,6 g (4,1 × $10^{-2}$ mole) de 3-aminocrotonate de 2-(2,2-diméthyl-1,3-dioxolanne-4-yl)-éthyle et 40 ml de tertiobutanol. Le solvant est évaporé et le produit brut obtenu est purifié par « flash chromatography » en éluant une première fois avec un mélange toluène-acétate d'éthyle-diisopropyléther (90 : 2 : 2) v/v puis une deuxième fois avec un mélange toluène-acétate d'éthyle diisopropyléther (90 : 1 : 1) v/v. On obtient 3,2 g (rendement : 22 %) de SR 1377 qui se présente sous la forme d'une huile.

Spectre de RMN ($CDCl_3$) δ (intensité, multiplicité) :
1,3 (3H,s) ; 1,38 (3H,s) ; 1,84 (2H,m) ; 2,37 (6H,s) ; 3,65 (3H,s) ; 3,5-4,27 (5H,m) ; 5,11 (1H,s) ; 5,98 (1H,s) ; 7,4-8,1 (4H,m).

Préparation IV

Obtention du 1,4-dihydro-2,6-diméthyl-4-(3-nitrophényl)-pyridine-3,5-dicarboxylate de méthyle et de (1,4-dioxa-spiro[4,4]nonane-2-yl)-méthyle (Exemple 5 ; N° de code : SR 1399)

On chauffe au reflux pendant 12 heures un mélange de 4,6 g ($1,79 \times 10^{-2}$ mole) de 3-nitrobenzylidène-acétylacétate de méthyle, 4,3 g ($1,78 \times 10^{-2}$ mole) de 3-aminocrotonate de (1,4-dioxa-spiro[4,4]nonane-2-yl)-méthyle et 45 ml de tertiobutanol. Le solvant est évaporé et le produit brut obtenu est purifié par « flash chromatography » en éluant avec un mélange hexane-acétone (9 : 1) v/v. On obtient 2,2 g (Rendement : 26 %) de SR 1399 qui se présente sous forme de mousse jaune.
Spectre de RMN ($CDCl_3$) δ (intensité, multiplicité) :
1,7 (8H,s) ; 2,37 (6H,s) ; 3,65 (3H,s) ; 3,65-5,12 (5H,m) ; 5,12 (1H,s) ; 5,87 (1H,s) ; 7,27-8,09 (4H,m).
En suivant le mode opératoire donné ci-dessus on obtient les composés suivants :

Exemple 3 (SR 1344), F = 119 °C
Spectre de RMN ($CDCl_3$) δ (intensité, multiplicité) :
2,37 (6H,s) ; 3,6 (3H,s) ; 3,6-4,24 (5H,m) ; 4,87 (1H,s) ; 4,99 (1H,s) ; 5,11 (1H,s) ; 6,15 (1H,s) ; 7,4-8,1 (4H,m).

Exemple 6 (SR 1411), F = 82 °C
Spectre de RMN ($CDCl_3$) δ (intensité, multiplicité) :
1,6 (3H,s), 1,65 (3H,s) ; 2,36-2,38 (6H, double singulet) ; 2,95 (2H,m) ; 3,66 (3H,s) ; 4,2-4,37 (3H,m) ; 5,84 (1H,s) ; 6,44 (1H,s) ; 7,38-8,11 (4H,m).

Exemple 7 (SR 1433), F = 50 °C
Spectre de RMN ($CDCl_3$) δ (intensité, multiplicité) :
1,6 (3H, 2 singulets) ; 2,37 (6H, 2 singulets) ; 3,6 (3H,s) ; 3,5-4,2 (5H,m) ; 5,05 (1H,m) ; 6,04 (1H,m) ; 7,2-7,7 (7H,m) ; 7,9-8,1 (2H,m).

Exemple 8 (SR 1457), F = 75 °C
Spectre de RMN ($CDCl_3$) δ (intensité, multiplicité) :
2,33 (6H, 2 singulets) ; 3,64 (3H,s) ; 3,5-4,2 (5H,m) ; 5,1 (1H,s) ; 5,9 (1H,s) ; 7,2-7,7 (12H,m) ; 7,8-8,1 (2H,m).

Exemple 9. F = 50 °C
Spectre de RMN ($CDCl_3$) δ (intensité, multiplicité) :
1,4-1,85 (10H,m) ; 2,38 (6H,s) ; 2,84 (2H,m) ; 3,65 (3H,s) ; 4,2 (3H,m) ; 5,13 (1H,s) ; 5,80 (1H,s) ; 7,2-7,7 (2H,m) ; 8,0-8,1 (2H,m).

Exemple 10 : F = 50 °C
Spectre de RMN ($CDCl_3$) δ (intensité, multiplicité) :
1,5-1,8 (12H,m) ; 2,36 (6H,s) ; 3,65 (3H,s) ; 3,5-4,2 (5H,m) ; 5,12 (1H,s) ; 5,96 (1H,s) ; 7,2-7,7 (2H,m) ; 8,0-8,1 (2H,m).

Exemple 11. F = 50 °C
Spectre de RMN ($CDCl_3$) δ (intensité, multiplicité) :
1,74 (3H,m) ; 2,35 (6H,m) ; 3,66 (3H,s) ; 3,5-4,2 (5H,m) ; 5,1 (1H,m) ; 5,88 (1H,m) ; 7,2-7,7 (7H,m) ; 7,9-8,1 (2H,m).

Exemple 12
Spectre de RMN ($CDCl_3$) δ (intensité, multiplicité) :
2,22-2,34-2,37-2,39 (6H, 4 singulets) ; 3,5-4,6 (8H,m dont 1 singulet à 3,66) ; 4,96-5,04-5,14 (1H,3 singulets) ; 6,0 (1H,s) ; 7,1-8,06 (9H,m).

Exemple 13 F = 113 °C
Spectre de RMN ($CDCl_3$) δ (intensité, multiplicité) :
2,37 (6H,s) ; 3,4-4,4 (8H,m) ; 5,1 (1H,s) ; 5,9 (1H,s) ; 7,3-8,07 (4H,m).

Exemple 14
Spectre de RMN ($CDCl_3$) δ (intensité, multiplicité) :
2,34-2,38 (6H,2 singulets) ; 3,64-3,6 (3H,2 singulets) ; 3,5-4,4 (5H,m) ; 5,11-5,14 (1H,2 singulets) ; 5,8-5,88-5,9 (1H, 3 singulets) ; 6,1 (1H,s) ; 7,2-8,1 (9H,m).

On a résumé ci-après les résultats des essais pharmacologiques qui ont été entrepris avec les composés selon l'invention, d'une part, et avec des produits antérieurement connus pour comparaison, d'autre part.

I. Effet relaxant sur aorte isolée de lapin contractée par KCl

Les aortes thoraciques de lapins mâles sont prélevées, découpées en bandelettes et mises sous tension dans un milieu de survie oxygéné. Les contractions de ces artères, dont l'amplitude est mesurée à l'aide d'un myographe, sont induites par une solution contenant KCl à 127 mM [R. Towart et al, Arzneim.-Forsch./Drug Res. 32, 338-346, (1982)]. Les composés étudiés sont dissous dans un mélange éthanol anhydre-eau bidistillée (1 : 1) v/v, le pourcentage d'éthanol dans les bains étant, en fin d'expérience, inférieur ou égal à 0,3 %. Lorsque le plateau de contraction est établi, le composé est ajouté dans le milieu à doses croissantes toutes les 15 minutes. A titre d'exemples, les doses relaxant de 50 % les artères précontractées (DE-50) sont données dans le tableau, IV comparativement à la nicardipine et à la nifédipine.

Dans cette expérience mettant en évidence les produits bloquant l'entrée de calcium dans le muscle lisse artériel les composés de l'invention sont actifs, dès lors qu'ils présentent une DE-50 du même ordre de grandeur que celle de la nicardipine et la nifédipine.

II. Activité antihypertensive

1. Administration orale

Des rats spontanément hypertendus âgés de 16 à 20 semaines dont utilisés. La pression artérielle systolique est mesurée par pléthysmographie et la fréquence cardiaque est obtenue à partir du tracé de pression. Les composés étudiés sont administrés par voie orale. Les résultats obtenus sont consignés dans le tableau V ci-après.

2. Administration intraveineuse

Des rats spontanément hypertendus âgés de 16 à 20 semaines sont utilisés. Sous anesthésie au diéthyléther, une canule est mise en place dans l'artère de la queue pour mesure de la pression artérielle et un cathéter dans la veine jugulaire droite pour administration des composés comme décrit précédemment (L. S. Watson and L.T. Ludden, New antihypertensive Drugs, p. 87-96, Spectrum Publication, Inc., 1976).

A titre d'exemple, on a représenté au moyen des figures annexées, la variation de la pression artérielle chez le rat spontanément hypertendu et conscient, après administration par voie intraveineuse d'une dose unique des composés des exemples 2, 7, 8 et 11, de la nicardipine et de la nifédipine. Chaque courbe C est relative au groupe témoin ne recevant que le véhicule, et chaque courbe T au groupe traité [n(nombre d'animaux par groupe) : 4-7]. Lesdits groupes sont comparés soit par analyse de variance lorsque les variances sont homogènes (test d'homocédasticité de Bartlett), soit par le test non paramétrique de Kruskall-Wallis (les résultats statistiquement significatifs étant représentés comme suit :* pour $p<0{,}05$ ; ** pour $p<0{,}01$ ; et *** pour $p<0{,}001$).

Les figures 1-6 concernent la variation de la pression artérielle systolique exprimée en mmHg (1 mmHg correspond approximativement à $1{,}333 \times 10^2$ Pa) en fonction du temps exprimé en minutes, et les figures 1a-6a la variation de la pression artérielle diastolique également exprimée en mmHg en fonction du temps exprimé en minutes, pour les groupes traités (courbes T) par rapport aux groupes témoins correspondants (courbes C), les doses de produits ayant été administrées étant les suivantes :

Figures 1 et 1a : nifédipine à 300 µg/kg i.v.,
Figures 2 et 2a : nicardipine à 30 µg/kg i.v.,
Figures 3 et 3a : exemple 2 à 30 µg/kg i.v.,
Figures 4 et 4a : exemple 7 à 100 µg/kg i.v.,
Figures 5 et 5a : exemple 8 à 300 µg/kg i.v., et
Figures 6 et 6a : exemple 11 à 30 et 100 µg/kg i.v. (courbes $T_1$ et $T_2$).

Le composé de l'exemple 2 entraîne une chute des pressions systolique et diastolique plus importante que la nicardipine à même dose et que la nifédipine à une dose 10 fois supérieure. La durée de l'effet antihypertenseur des composés des exemples 2, 7, 8 et 11 est notablement plus élevée que celle de la nicardipine et de la nifédipine.

III. Effets hémodynamiques chez le chien anesthésié

Des chiens bâtards sont anesthésiés au pentobarbital sodique (25 mg/kg i.v.). Chaque animal étant placé sous respiration artificielle, un cathéter est introduit dans l'aorte pour mesure de la pression artérielle. Les débits des artères, aorte (D. Aor.), fémorale droite (D. Fem.) et coronaire gauche (branche

descendante) (D. Cor.) sont mesurés en continu à l'aide de bagues électromagnétiques. Les résistances périphériques totales (R. P. T.) sont également calculées.

Les composés sont administrés par voie intraduodénale (i. d.) à des doses choisies pour entraîner une baisse de pression artérielle diastolique (P. A. D.) d'environ 40 % (de 34,5 à 44,2 %). Les résultats obtenus sont consignés dans le tableau VI.

On constate que les composés des exemples 1 et 2 entraînent une baisse des résistances périphériques totales plus forte que la nicardipine et la nifédipine, la durée de cet effet étant considérablement plus importante. Les débits cardiaque, fémoral et coronaire sont plus durablement et plus fortement augmentés avec les composés des exemples 1 et 2 comparativement à la nicardipine et à la nifédipine.

IV. Toxicité aiguë.

La DL-50 a été déterminée par voie intraveineuse chez le rat Wistar mâle de 300 g.

Les DL-50 sont respectivement de 11,8 mg/kg pour la nicardipine, 13,2 mg/kg pour la nifédipine, 22,5 mg/kg pour le composé de l'exemple 1, 27,9 mg/kg pour le composé de l'exemple 2, 34,2 mg/kg pour le composé de l'exemple 6 et 17,8 mg/kg pour le composé de l'exemple 7, 44,4 mg/kg pour le composé de l'exemple 8, 38,9 mg/kg pour le composé de l'exemple 11.

V. Conclusions

Les résultats des essais sus-visés mettent en évidence que les composés selon l'invention, et en particulier ceux des exemples 1, 2, 7, 8 et 11 se distinguent des produits antérieurement connus

— par un effet antihypertenseur de plus longue durée,

— par une augmentation en volume et en durée des débits cardiaque, fémoral et coronaire,

— par une diminution importante de la postcharge cardiaque eu égard à l'abaissement des résistances périphériques totales,

— par une toxicité aiguë inférieure.

Les composés selon l'invention qui sont des agents vasodilatateurs remarquables sont particulièrement utiles dans le traitement de l'hypertension, d'une part, et dans la prévention et le traitement des troubles circulatoires périphériques, cérébraux et coronaires, d'autre part. En clinique on a constaté que la particularité d'accroître le débit cardiaque et d'abaisser fortement les résistances périphériques, confère aux produits selon l'invention un grand intérêt notamment dans le traitement de l'insuffisance cardiaque ou de l'hypertension, plus particulièrement chez le sujet âgé.

Les produits selon l'invention peuvent être administrés à l'homme notamment par voie orale à la dose de 0,005 à 10 mg/kg ou par voie intraveineuse à la dose de 1 à 300 µg/kg.

Selon l'invention on préconise également l'utilisation d'une substance choisie parmi l'ensemble des dérivés de formule I, leurs isomères optiques et leurs diastéréoisomères, pour l'obtention d'un médicament vasodilatateur destiné à une utilisation en thérapeutique humaine vis-à-vis de l'hypertension et de l'insuffisance cardiaque.

(Voir Tableau IV page 16)

Tableau IV

Effet relaxant sur l'aorte de lapin dépolarisée par KCl

| PRODUIT | DE-50 (M) |
|---|---|
| Ex 1 | $3,1 \times 10^{-8}$ |
| Ex 2 | $5,0 \times 10^{-8}$ |
| Ex 3 | $4,3 \times 10^{-8}$ |
| Ex 4 | $3,2 \times 10^{-8}$ |
| Ex 6 | $3,5 \times 10^{-8}$ |
| Ex 8 | $> 5 \times 10^{-6}$ |
| Ex 11 | $> 5 \times 10^{-6}$ |
| Ex 12 | $5 \times 10^{-6}$ |
| Ex 13 | $> 5 \times 10^{-6}$ |
| Ex 14 | $> 5 \times 10^{-6}$ |
| CP 1 (a) | $2,5 \times 10^{-7}$ |
| nicardipine | $2,6 \times 10^{-8}$ |
| nifédipine | $2,2 \times 10^{-8}$ |

Note

(a) Produit de comparaison (décrit à l'exemple 1 de FR A 2 514 761) à savoir le 2,6-diméthyl-4 (3-nitrophényl)-1,4-dihydropyridine-3,5-dicarboxylate de méthyle et de 2,2-éthylènedioxypropyle.

(Voir Tableau V page 17)

Tableau V

Activité antihypertensive chez le rat spontanément hypertendu - voie orale -

| PRODUIT | DOSE (mg/kg) | % de variation | |
|---|---|---|---|
| | | Pression art. systolique * | Fréquence cardiaque * |
| Ex 1 | 30 | -28,4 ± 7,3(8 à 16) | +16,4 ± 9,6(2 à 4 ) |
| Ex 2 | 30 | -45,4 ± 6,3(> 24) | +24,5 ± 9,7(8 à 16) |
| Ex 5 | 30 | -40,4 ± 9,4(4 à 6) | +19,3 ± 9,1 (4) |
| Ex 6 | 30 | -35,1 ± 9,2(4 à 6) | +21,4 ± 12,8(1 à 2) |
| Ex 7 | 30 | -40,5 ± 5,1 (> 24) | +36,4 ± 12,5(8 à 24) |
| Ex 8 | 30 | -37,0 ± 9,0 (> 24) | +12,4 ± 11,3(2 à 4) |
| Ex 11 | 30 | -54,0 ± 5,9 (> 24) | +34,0 ± 17,0(8 à 24) |
| Ex 12 | 10 | -41,4 ± 10,0(> 24) | +31,2 ± 13,8(8 à 24) |
| Ex 13 | 30 | -31,6 ± 8,7 (> 24) | +25,0 ± 11,2( 6 à 8) |
| Ex 14 | 30 | -58,4 ± 32,9(> 24) | +19,9 ± 14,8(> 24) |
| CP 1(a) | 30 | -11,6 ± 6,0 (0) | 0 |
| nicardipine | 30 | -46,2 ± 6,8(8 à 24) | +21,3 ± 10,8 (8 à 24) |
| nifédipine | 30 | -35,2 ± 4,7(8 à 24) | +35,4 ± 15 (4 à 6) |

Notes :

* : entre parenthèses on a indiqué la durée de l'effet en heures

(a) Produit de comparaison (décrit à l'exemple 1 de FR A2514761) à savoir le 2,6-diméthyl-4-(3-nitrophényl)-1,4-dihydropyridine-3,5-dicarboxylate de méthyle et de 2,2-éthylènedioxypropyle.

(Voir Tableau VI page 18)

Tableau VI

Effets hémodynamiques chez le chien anesthésié
- voie intraduodénale -

| Produit | dose (mg / kg) | % de variation (a) (b) | | | | |
|---|---|---|---|---|---|---|
| | | P.A.D. | R.P.T. | D.Aor. | D. Fem. | D.Cor. |
| Ex 1 | 0,5 | - 42,6(120) | - 65(300) | + 124(120) | + 114(270) | + 73,6(NS) |
| Ex 2 | 0,5 | - 41,2(240) | - 53,4(300) | + 40,2(300) | + 180,5(300) | + 89,5(300) |
| Nifédipine | 0,35 | - 44,2(240) | - 48,6(180) | + 20,5(NS) | + 76,4(10) | + 66,4(NS) |
| Nicardipine | 0,5 | - 34,5(150) | - 40,5(90) | + 25 (NS) | + 70,6(NS) | + 62,2(NS) |

Notes :
(a) : durée de l'effet exprimée en minutes donnée entre parenthèses.
(b) : la mention NS (non significatif) indique que l'effet mesuré n'a pas varié de façon statistiquement différente comparativement à un groupe de chiens ayant reçu le véhicule. Les groupes ont été comparés soit par analyse de variance lorsque les variances étaient homogènes (test d'homocédasticité de Bartlett) soit par le test non paramétrique de Kruskall-Wallis.

**Revendications** (pour les Etats contractants : BE, CH, DE, FR, GB, IT, LI, LU, NL, SE)

1. Dérivé ester hétérocyclique dissymétrique d'acide 1,4-dihydropyridine-3,5-dicarboxylique, caractérisé en ce qu'il est choisi parmi l'ensemble constitué par

(i) les esters d'acide 1,4-dihydro-2,6-diméthyl-pyridine-3,5-dicarboxylique de formule générale

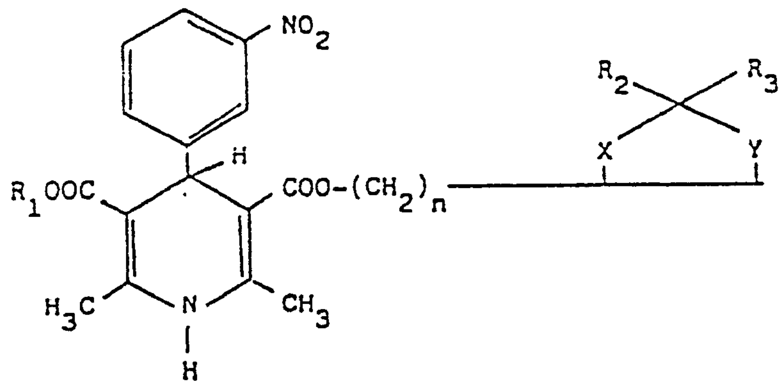

(I)

dans laquelle

$R_1$ est un groupe alkyle en $C_1$-$C_4$ ;

X et Y identiques ou différents représentent chacun O ou S, un au moins des X et Y étant différent de S ;

n est un nombre entier valant 1 ou 2 ;

$R_2$ et $R_3$, identiques ou différents, représentent chacun l'atome d'hydrogène, le groupe méthyle, le groupe trifluorométhyle, le groupe phényle ou un groupe halogénophényle, $R_2$ et $R_3$ considérés ensemble pouvant former avec l'atome de carbone de l'hétérocycle auquel ils sont liés un groupe spirocycloaliphatique de 5 à 7 sommets ; et

(ii) leurs isomères optiques et diastéréoisomères

2. Dérivé selon la revendication 1, caractérisé en ce que $R_2$ et $R_3$ forment avec l'atome de carbone du groupe hétérocyclique auquel ils sont liés un reste cyclopentyle, cyclohexyle ou cycloheptyle.

3. Dérivé selon la revendication 1, caractérisé en ce que $R_2$ et $R_3$, identiques ou différents, représentent chacun H, $CH_3$, $CF_3C_6H_5$ ou 2,4-dichlorophényle.

4. 1,4-Dihydro-2,6-diméthyl-4-(3-nitrophényl)-pyridine-3,5-dicarboxylate de méthyle et de (2,2-diméthyl-1,3-dioxolanne-4-yl)-méthyle.

5. 1,4-Dihydro-2,6-diméthyl-4-(3-nitrophényl)-pyridine-3,5-dicarboxylate de méthyle et de (1,4-dioxaspiro [4,5] décane-2-yl)-méthyle.

6. 1,4-Dihydro-2,6-diméthyl-4-(3-nitrophényl)-pyridine-3,5-dicarboxylate de méthyle et de (2,2-diphényl-1,3-dioxolanne-4-yl)-méthyle.

7. Composition thérapeutique caractérisée en ce qu'elle renferme, en association avec un excipient physiologiquement acceptable, au moins un dérivé de formule I selon la revendication 1 ou l'un de ses isomères énantiomères ou diastéréoisomères.

8. Procédé de préparation d'un composé de formule I selon la revendication 1, caractérisé en ce que l'on fait réagir un nitrobenzylidène-acétoacétate de formule

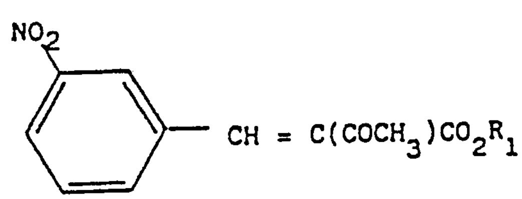

(II)

où $R_1$ est défini comme indiqué ci-dessus, avec un aminocrotonate de formule

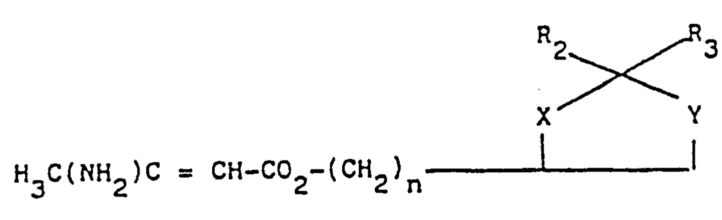

(III)

où n, X, Y, $R_2$ et $R_3$ sont définis comme indiqué ci-dessus.

9. Procédé selon la revendication 8, pour la préparation d'un aminocrotonate de formule III caractérisé en ce que

1) on fait réagir un alcool de formule

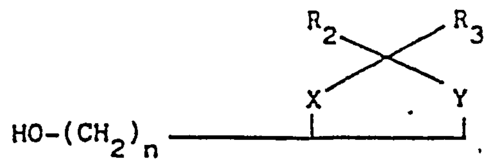

(IV)

où n, X, Y, $R_2$ et $R_3$ sont définis comme indiqué ci-dessus, avec le 5-acétyl-2,2-diméthyl-4,6-dioxo-1,3-dioxanne de formule

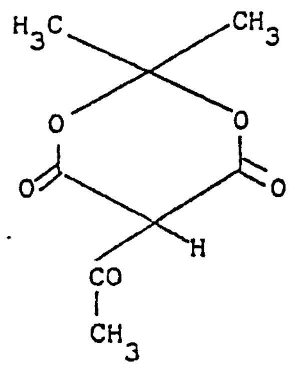

(V)

dans un solvant aromatique à une température comprise entre la température ambiante (15-20 °C) et la température de reflux du milieu réactionnel, pendant 1 à 24 h, pour obtenir un céto-ester de formule

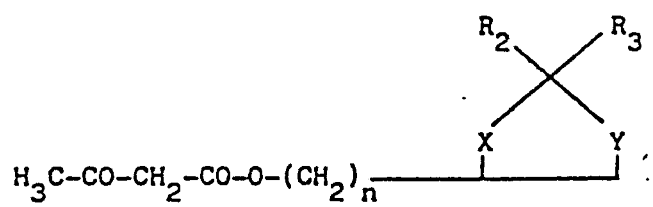

(VI)

2) on fait réagir le dérivé de formule VI ainsi obtenu avec $NH_3$ dans un solvant chloré pendant 1 à 20 h.

10. Produit intermédiaire intervenant dans la synthèse de composés de formule I selon la revendication 1, caractérisé en ce qu'il est choisi parmi l'ensemble constitué par

(a) les céto-esters de formule

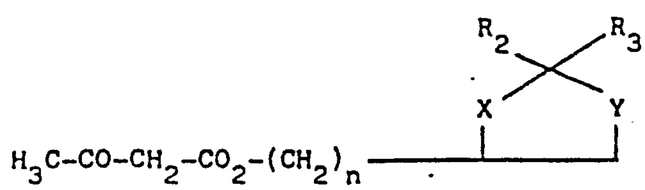

(VI)

et

dans laquelle n, $R_2$, $R_3$, X et Y sont définis comme indiqué ci-dessus dans la revendication 1 avec la condition supplémentaire que n est différent de 1 quand on a simultanément (i) $R_2 = R_3 = CH_3$ et (/ii) X = Y = 0, et

(b) les aminocrotonates de formule

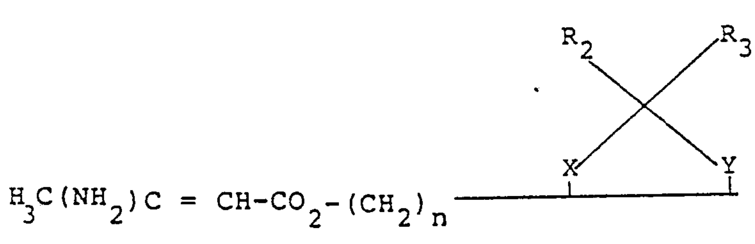

(III)

dans laquelle n, $R_2$, $R_3$, X et Y sont définis comme indiqué ci-dessus dans la revendication 1.

**Revendications** (pour l'Etat contractant AT)

1. Procédé de préparation d'un dérivé ester hétérocyclique dissymétrique d'acide 1,4-dihydropiridine-3,5-dicarboxylique appartenant à l'ensemble des esters d'acide 1,4-dihydro-2,6-diméthyl-pyridine-3,5-dicarboxylique de formule générale

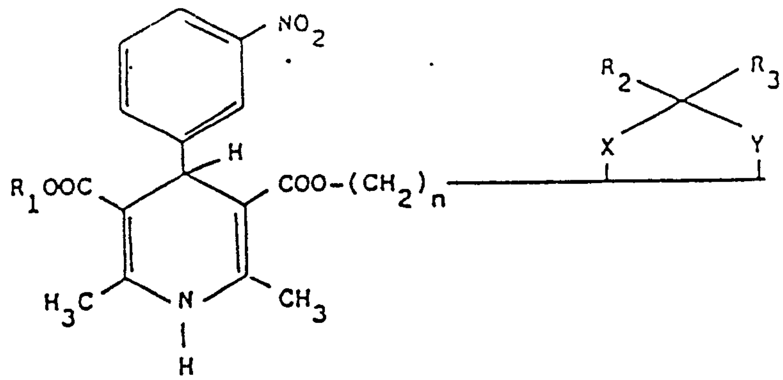

(I)

dans laquelle

$R_1$ est un groupe alkyle en $C_1$-$C_4$ ;

X et Y identiques ou différents représentent chacun O ou S, un au moins des X et Y étant différent de S ;

n est un nombre entier valant 1 ou 2 ;

$R_2$ et $R_3$, identiques ou différents, représentent chacun l'atome d'hydrogène, le groupe méthyle, le groupe trifluorométhyle, le groupe phényle ou un groupe halogénophényle, $R_2$ et $R_3$ considérés ensemble pouvant former avec l'atome de carbone de l'hétérocycle auquel ils sont liés un groupe spirocycloaliphatique de 5 à 7 sommets ; et ses isomères optiques et diastéréoisomères ;

ledit procédé étant caractérisé en ce que l'on fait réagir un nitrobenzylidène-acétoacétate de formule

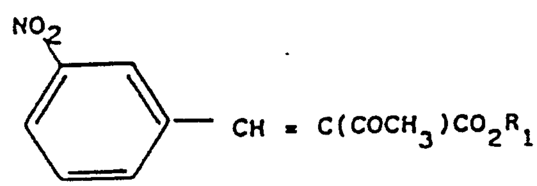

(II)

où $R_1$ est défini comme indiqué ci-dessus, avec un aminocrotonate de formule

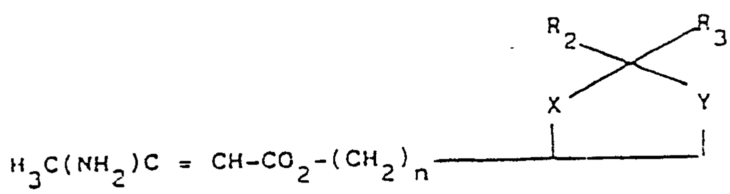

(III)

où n, X, Y, $R_2$ et $R_3$ sont définis comme indiqué ci-dessus.

2. Procédé selon la revendication 1, caractérisé en ce que, dans le composé III et le composé I, $R_2$ et $R_3$ forment avec l'atome de carbone du groupe hétérocyclique auquel ils sont liés un reste cyclopentyle, cyclohexyle ou cycloheptyle.

3. Procédé selon la revendication 1, caractérisé en ce que, dans le composé III et le composé I, $R_2$ et $R_3$, identiques ou différents, représentent chacun H, $CH_3$, $CF_3$, $C_6H_5$ ou 2,4-dichlorophényle.

4. Procédé selon la revendication 1, caractérisé en ce que $R_1$, $R_2$ et $R_3$ représentent chacun $CH_3$, n est 1, et X et Y représentent chacun O.

5. Procédé selon la revendication 1, caractérisé en ce que $R_1$ est $CH_3$, n est 1 et le groupe

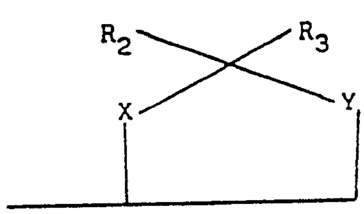

est le groupe 1,4-dioxa-spiro [4,5] décane-2-yle.

6. Procédé selon la revendication 1, caractérisé en ce que $R_1$ est $CH_3$, n est 1, $R_2$ et $R_3$ représentent chacun $C_6H_5$, et X et Y représentent chacun O.

7. Procédé selon la revendication 1, pour la préparation d'un aminocrotonate de formule III caractérisé en ce que

1) on fait réagir un alcool de formule

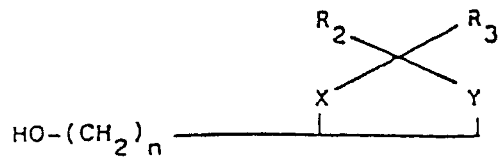

(IV)

où n, X, Y, $R_2$ et $R_3$ sont définis comme indiqué ci-dessus, avec le 5-acétyl-2,2-diméthyl-4,6-dioxo-1,3-dioxanne de formule

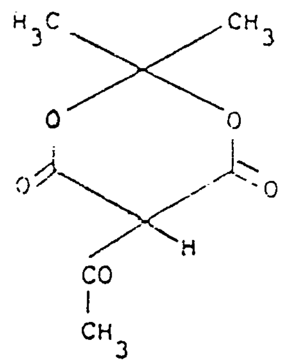

(V)

dans un solvant aromatique à une température comprise entre la température ambiante (15-20 °C) et la température de reflux du milieu réactionnel, pendant 1 à 24 h, pour obtenir un céto-ester de formule

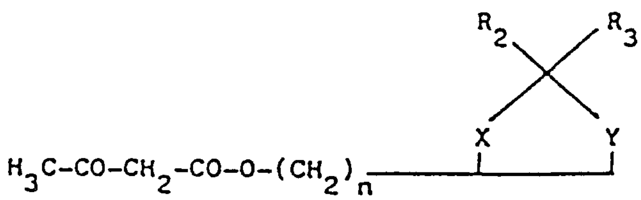

(VI)

2) on fait réagir le dérivé de formule VI ainsi obtenu avec $NH_3$ dans un solvant chloré pendant 1 à 20 h.

**Claims** (for the Contracting States : BE, CH, DE, FR, GB, IT, LI, LU, NL, SE)

1. An asymmetric heterocyclic ester derivative of a 1,4-dihydropyridine-3,5-dicarboxylic acid, selected from the group comprising :

(i) the 1,4-dihydro-2,6-dimethylpyridine-3,5-dicarboxylic acid esters of the general formula :

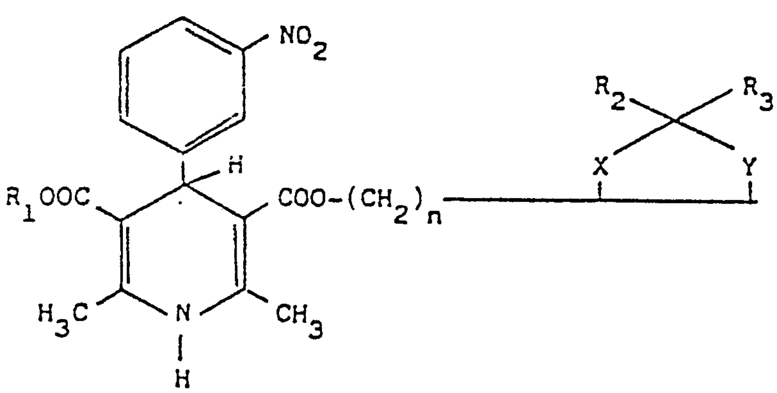

(I)

wherein

$R^1$ is a $C_1$-$C_4$-alkyl group ;

X and Y, which are identical or different, each represent O or S, at least one of the symbols X and Y being different from S ;

n is an integer equal to 1 or 2 ; and

$R_2$ and $R_3$, which are identical or different, each represent the hydrogen atom, the methyl group, the trifluoromethyl group, the phenyl group or a halogenophenyl group, it being possible for $R_2$ and $R_3$, taken together, to form a 5-membered to 7-membered spirocycloaliphatic group with the carbon atom of the heterocycle to which they are bonded ; and

(ii) their optical isomers and diastereoisomers.

2. A derivative according to claim 1, wherein $R_2$ and $R_3$ form a cyclopentyl, cyclohexyl or cycloheptyl radical with the carbon atom of the heterocyclic group to which they are bonded.

3. A derivative according to claim 1, wherein $R_2$ and $R_3$, which are identical or different, each represent H, $CH_3$, $CF_3$, $C_6H_5$ or 2,4-dichlorophenyl.

4. Methyl (2,2-dimethyl-1,3-dioxolan-4-yl)methyl 1,4-dihydro-2,6-dimethyl-4-(3-nitrophenyl)pyridine-3,5-dicarboxylate.

5. Methyl (1,4-dioxaspiro [4,5] decan-2-yl)methyl 1,4-dihydro-2,6-dimethyl-4-(3-nitrophenyl) pyridine-3,5-dicarboxylate.

6. Methyl (2,2-diphenyl-1,3-dioxolan-4-yl)methyl 1,4-dihydro-2,6-dimethyl-4-(3-nitrophenyl)pyridine-3,5-dicarboxylate.

7. A pharmaceutical composition comprising, in association with a physiologically acceptable excipient, a pharmaceutically effective quantity of a derivative of the formula I according to claim 1 or one of its optical isomers or diastereoisomers.

8. A method for the preparation of a derivative of the formula I according to claim 1, which comprises reacting a nitrobenzylideneacetoacetate of the formula :

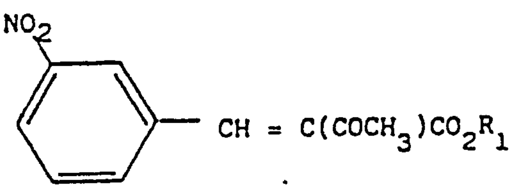

(II)

wherein $R_1$ is defined as indicated above, with an aminocrotonate of the formula :

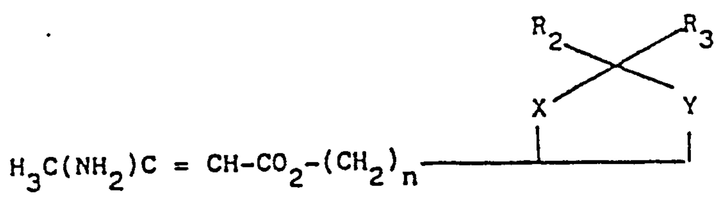

(III)

wherein n, X, Y, $R_2$ and $R_3$ are defined as indicated above.

9. The method according to claim 8 for the preparation of an aminocrotonate of the formula III, which comprises :

1) reaction of an alcohol of the formula :

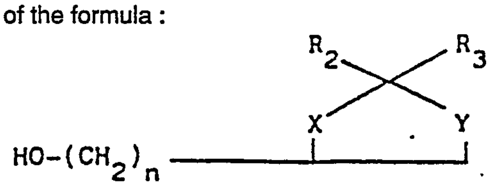

(IV)

wherein n, X, Y, $R_2$ and $R_3$ are defined as indicated above, with 5-acetyl-2,2-dimethyl-4,6-dioxo-1,3-dioxane of the formula :

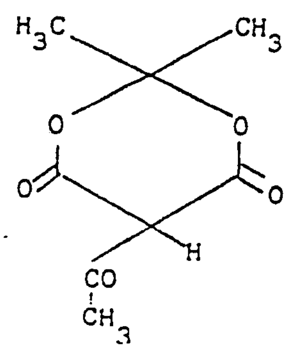

(V)

in an aromatic solvent, at a temperature between room temperature (15-20 °C) and the reflux temperature of the reaction medium, for 1 to 24 hours, to give a keto-ester of the formula :

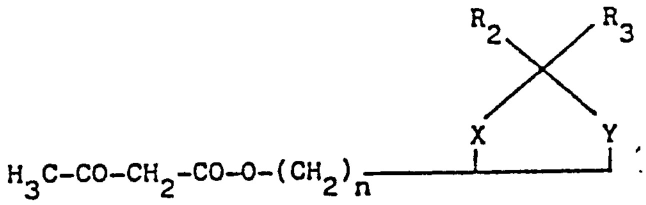

(VI)

and

2) reaction of the resulting derivative of the formula VI with $NH_3$ in a chlorinated solvent for 1 to 20 hours.

10. An intermediate useful in the preparation of a derivative of the formula I according to claim 1, which is selected from the group comprising :

(a) the keto-esters of the formula :

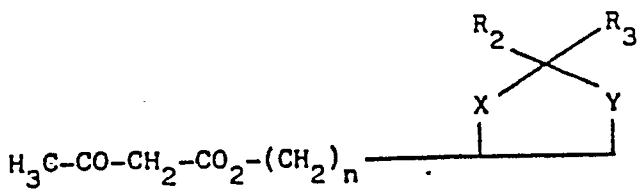

(VI)

wherein n, $R_2$, $R_3$, X and Y are defined as indicated above in claim 1, with the supplemental proviso that n is different from 1, when simultaneously (i) $R_2=R_3=CH_3$, and (ii) X=Y=O ; and

(b) the aminocrotonates of the formula :

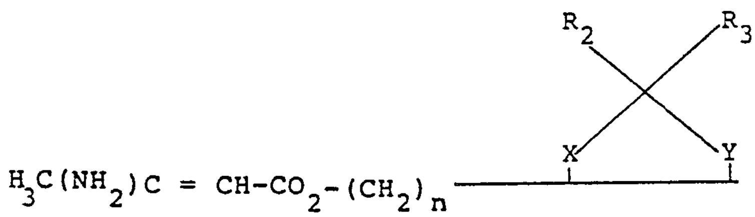

(III)

wherein n, $R_2$, $R_3$, X and Y are defined as indicated above in claim 1.

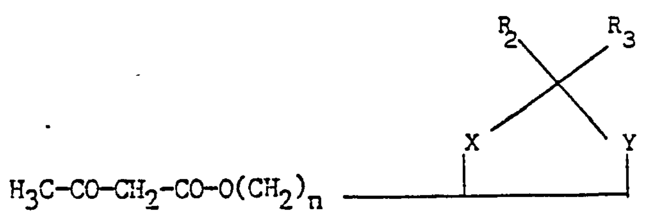

(III)

**Claims** (for the Contracting State AT)

1. A method for the preparation of an asymmetric heterocyclic ester derivative of a 1,4-dihydropyridine-3,5-dicarboxylic acid belonging to the family of 1,4-dihydro-2,6-dimethylpyridine-3,5-dicarboxylic acid esters of the general formula :

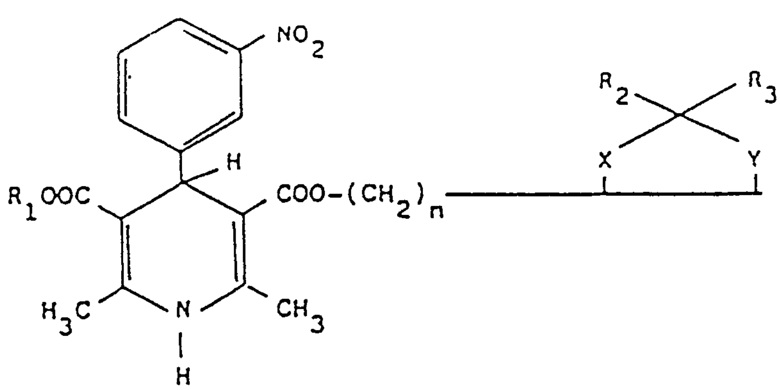

(I)

wherein

$R^1$ is a $C_1$-$C_4$-alkyl group ;

X and Y, which are identical or different, each represent O or S, at least one of the symbols X and Y being different from S ;

n is an integer equal to 1 or 2 ; and

$R_2$ and $R_3$, which are identical or different, each represent the hydrogen atom, the methyl group, the trifluoromethyl group, the phenyl group or a halogenophenyl group, it being possible for $R_2$ and $R_3$, taken together, to form a 5-membered to 7-membered spirocycloaliphatic group with the carbon atom of the heterocycle to which they are bonded ; their optical isomers and diastereoisomers,

said method comprising reacting a nitrobenzylideneacetoacetate of the formula :

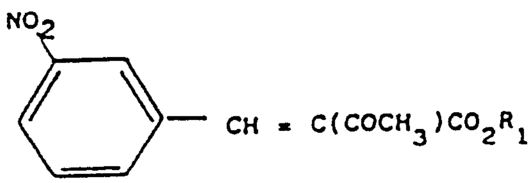

(II)

wherein $R_1$ is defined as indicated above, with an aminocrotonate of the formula :

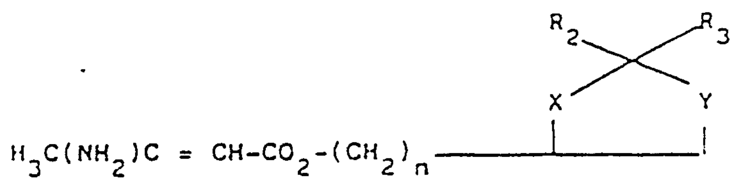

(III)

wherein n, X, Y, $R_2$ and $R_3$ are defined as indicated above.

2. The method according to claim 1, wherein in the compound of the formula III and in the compound of the formula I, $R_2$ and $R_3$ form a cyclopentyl, cyclohexyl or cycloheptyl radical with the carbon atom of the heterocyclic group to which they are bonded.

3. The method according to claim 1, wherein in the compound of the formula III and in the compound of the formula I, $R_2$ and $R_3$, which are identical or different, each represent H, $CH_3$, $CF_3$, $C_6H_5$ or 2,4-dichlorophenyl.

4. The method according to claim 1, wherein $R_1$, $R_2$ and $R_3$ represent each $CH_3$, n is 1, and, X and Y represent each O.

5. The method according to claim 1, wherein $R_1$ is $CH_3$, n is 1 and the group

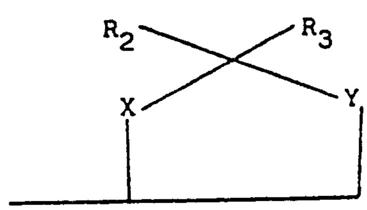

represents the 1,4-dioxa-spiro [4,5] decan-2-yl group.

6. The method according to claim 1, wherein $R_1$ is $CH_3$, $R_2$ and $R_3$ are each $C_6H_5$, and, X and Y are each O.

7. The method according to claim 1 for the preparation of an aminocrotonate of the formula III, which comprises :

1) reaction of an alcohol of the formula :

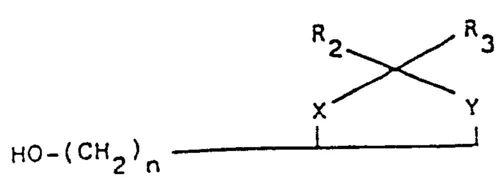

(IV)

wherein n, X, Y, $R_2$ and $R_3$ are defined as indicated above, with 5-acetyl-2,2-dimethyl-4,6-dioxo-1,3-dioxane of the formula :

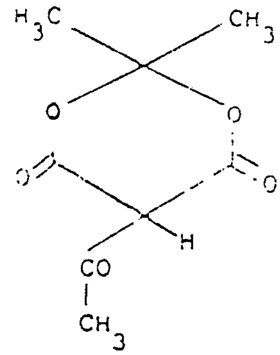

(V)

in an aromatic solvent at a temperature between room temperature (15-20 °C) and the reflux temperature of the reaction medium, for 1 to 24 hours, to give a keto-ester of the formula :

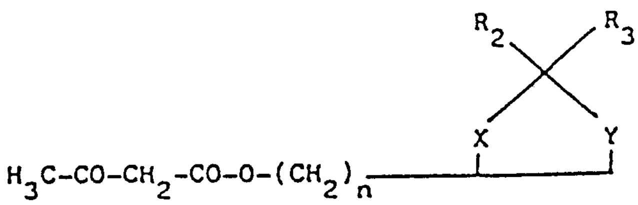

(VI)

and

2) reaction of the resulting derivative of the formula VI with $NH_3$ in a chlorinated solvent for 1 to 20 hours.

**Patentansprüche** (für die Vertragsstaaten : BE, CH, DE, FR, GB, IT, LI, LU, NL, SE)

1. Heterozyklisches unsymmetrisches Esterderivat von 1,4-Dihydropyridin-3,5-dicarboxylsäure, dadurch gekennzeichnet, daß es ausgewählt ist aus der Gruppe, bestehend aus

(i) den Estern von 1,4-Dihydro-2,6-dimethyl-pyridin-3,5-dicarboxylsäure der allgemeinen Formel

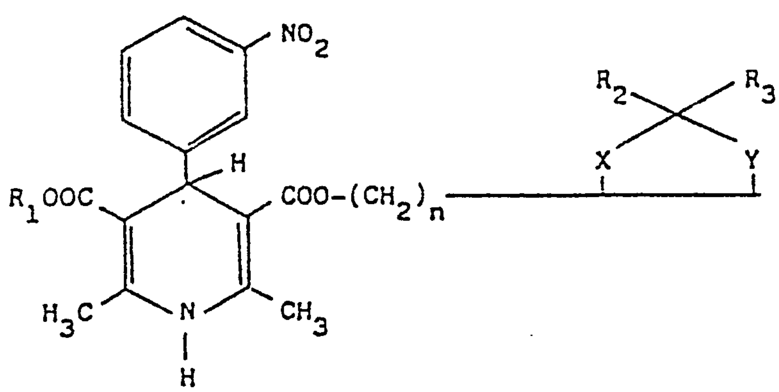

(I)

in welcher

$R_1$ eine Alkylgruppe mit $C_1$ bis $C_4$ ist ;

X und Y gleich oder verschieden sind und jeweils O oder S darstellen, wobei X und/oder Y von S verschieden sind/ist ;

n eine ganze Zahl vom Betrag 1 oder 2 ist ;

$R_2$ und $R_3$ gleich oder verschieden sind und jeweils das Wasserstoffatom, die Methylgruppe, die Trifluoromethylgruppe, die Phenylgruppe oder eine Halogenophenylgruppe darstellen, wobei $R_2$ und $R_3$ miteinander in Verbindung mit dem Kohlenstoffatom der heterozyklischen Verbindung, mit der sie verbunden sind, eine spirocycloaliphatische Gruppe mit 5 bis 7 Ecken bilden können ; und

(ii) ihren optischen Isomeren und Diastereoisomeren.

2. Derivat nach Anspruch 1, dadurch gekennzeichnet, daß $R_2$ und $R_3$ mit dem Kohlenstoffatom der heterozyklischen Gruppe, mit der sie verbunden sind, einen Cyclopentylrest, einen Cyclohexylrest oder einen Cycloheptylrest bilden.

3. Derivat nach Anspruch 1, dadurch gekennzeichnet, daß $R_2$ und $R_3$, die gleich oder verschieden sind, jeweils H, $CH_3$, $CF_3$, $C_6H_5$ oder 2,4-Dichlorophenyl darstellen.

4. 1,4-Dihydro-2,6-dimethyl-4-(3-nitrophenyl)-pyridin-3,5-dicarboxylat von Methyl und von (2,2-dimethyl-1,3-dioxolan-4-yl)-methyl.

5. 1,4-Dihydro-2,6-dimethyl-4-(3-nitrophenyl)-pyridin-3,5-dicarboxylat von Methyl und von (1,4-dioxaspiro [4,5] decan-2-yl)-methyl.

6. 1,4-Dihydro-2,6-dimethyl-4-(3-nitrophenyl)-pyridin-3,5-dicarboxylat von Methyl und von (2,2-diphenyl-1,3-dioxolan-4-yl)-methyl.

7. Therapeutische Zusammensetzung, dadurch gekennzeichnet, daß sie in Verbindung mit einer physiologisch annehmbaren Grundmasse mindestens ein Derivat der Formel I in Anspruch 1 oder eines von dessen enantiomeren Isomeren oder Diastereoisomeren umfaßt.

8. Verfahren zur Herstellung einer Verbindung der Formel I nach Anspruch 1, dadurch gekennzeichnet, daß ein Nitrobenzyliden-acetoacetat der Formel

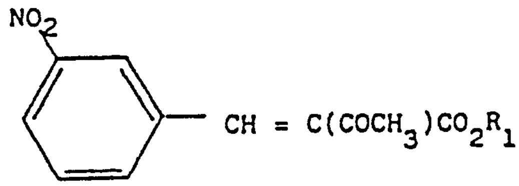

(II)

worin $R_1$ die oben angegebene Bedeutung hat, umgesetzt wird mit einem Aminocarbonat der Formel

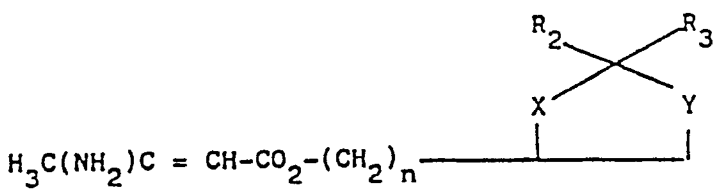

(III)

worin n, X, Y, $R_2$ und $R_3$ die oben angegebene Bedeutung haben.

9. Verfahren nach Anspruch 8 zur Herstellung eines Aminocrotonats der Formel III, dadurch gekennzeichnet, daß

1) ein Alkohol der Formel

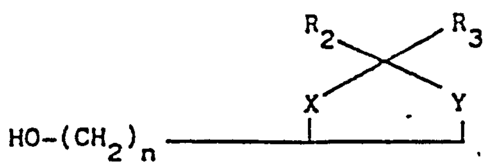

(IV)

worin n, X, Y, $R_2$ und $R_3$ die oben angegebene Bedeutung haben, umgesetzt wird mit 5-Acetyl-2,2-dimethyl-4,6-dioxo-1,3-dioxan der Formel

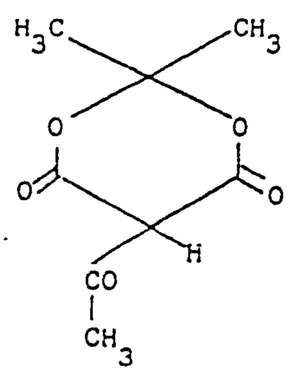

(V)

in einem aromatischen Lösungsmittel bei einer Temperatur, die zwischen der Raumtemperatur (15-20 °C) und der Refluxtemperatur des Reaktionsmediums liegt, während 1 bis 24 Std., um einen Ketoester der Formel

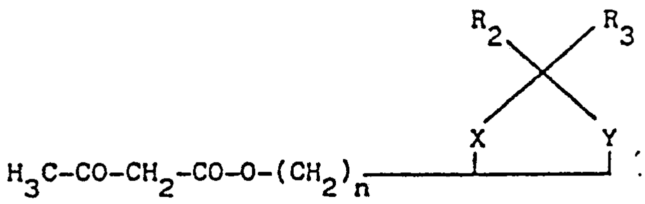

(VI)

zu erhalten, und

2) das so erhaltene Derivat von Formel VI während 1 bis 20 Std. in einem chlorierten Lösungsmittel mit $NH_3$ umgesetzt wird.

10. Zwischenprodukt, das bei der Synthese der Verbindung von Formel I nach Anspruch 1 auftritt, dadurch gekennzeichnet, daß es gewählt wird aus der Gruppe, bestehend aus

(a) den Ketoestern der Formel

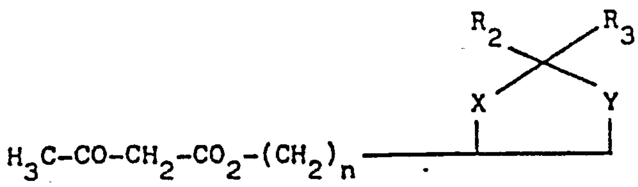

(VI)

worin n, $R_2$, $R_3$, X und Y die gleiche Bedeutung haben wie in Anspruch 1 angegeben, mit der zusätzlichen Bedingungen, daß n von 1 verschieden ist, wenn gleichzeitig gilt (i) $R_2=R_3=CH_3$ und (ii) X=Y=O, und

(b) den Aminocrotanaten der Formel

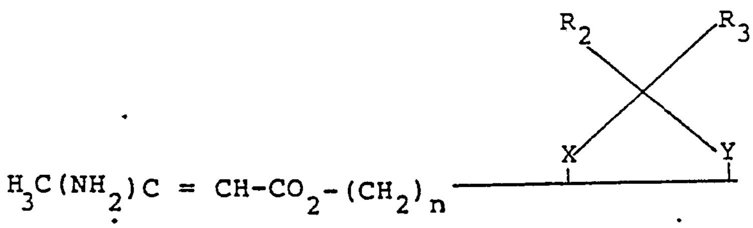

(III)

worin n, $R_2$, $R_3$, X und Y die gleiche Bedeutung haben, wie oben in Anspruch 1 angegeben.

**Patentansprüche** (für den Vertragsstaat AT)

1. Verfahren zur Herstellung eines heterozyklischen unsymmetrischen Esterderivats von 1,4-Dihydropyridin-3,5-dicarboxylsäure, das zu der Gruppe der 1,4-Dihydro-2,6-dimethyl-pyridin-3,5-dicarboxylsäureester der allgemeinen Formel

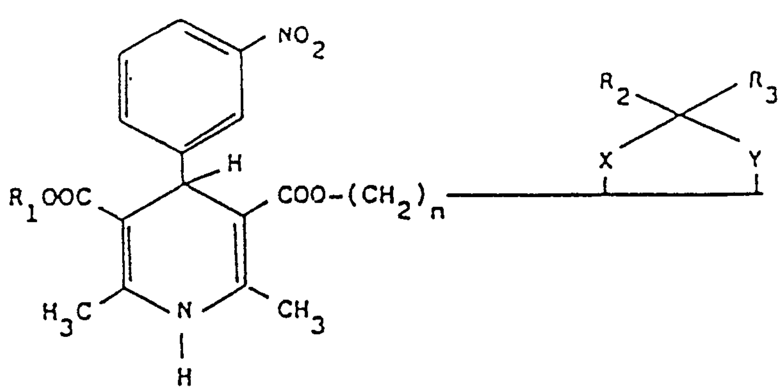

(I)

gehört, in welcher

$R_1$ eine Alkylgruppe mit $C_1$ bis $C_4$ ist ;

X und Y gleich oder verschieden sind und jeweils O oder S darstellen, wobei X und/oder Y von S verschieden sind/ist ;

n eine ganze Zahl vom Betrag 1 oder 2 ist ;

$R_2$ und $R_3$ gleich oder verschieden sind und jeweils das Wasserstoffatom, die Methylgruppe, die Trifluoromethylgruppe, die Phenylgruppe oder eine Halogenophenylgruppe darstellen, wobei $R_2$ und $R_3$ miteinander in Verbindung mit dem Kohlenstoffatom der heterozyklischen Verbindung, mit der sie verbunden sind, eine spirocycloaliphatische Gruppe mit 5 bis 7 Ecken bilden können ; sowie deren optische Isomere und Diastereoisomere ; welches Verfahren dadurch gekennzeichnet ist,
daß ein Nitrobenzyliden-acetoacetat der Formel

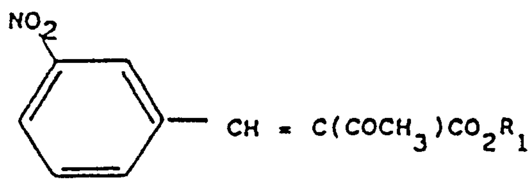

(II)

worin $R_1$ die oben angegebene Bedeutung hat, umgesetzt wird mit einem Aminocrotonat der Formel

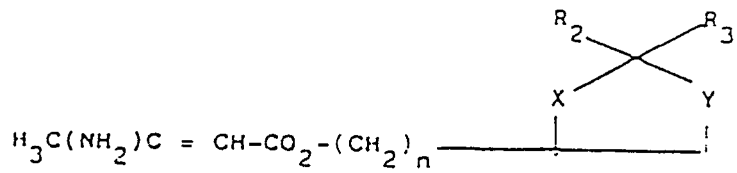

(III)

worin n, X, Y, $R_2$ und $R_3$ die oben angegebene Bedeutung haben.

2. Verfahren nach Anspruch 1, dadurch gekennzeichnet, daß in der Verbindung III und der Verbindung I $R_2$ und $R_3$ mit dem Kohlenstoffatom der heterozyklischen Gruppe, mit der sie verbunden sind, einen Cyclopentylrest, einen Cyclohexylrest oder einen Cycloheptylrest bilden.

3. Verfahren nach Anspruch 1, dadurch gekennzeichnet, daß in der Verbindung III und der Verbindung I $R_2$ und $R_3$, die gleich oder verschieden sind, jeweils H, $CH_3$, $CF_3$, $C_6H_5$ oder 2,4-Dichlorphenyl darstellen.

4. Verfahren nach Anspruch 1, dadurch gekennzeichnet, daß $R_1$, $R_2$ und $R_3$ jeweils $CH_3$ darstellen, daß n = 1 und daß X und Y jeweils O darstellen.

5. Verfahren nach Anspruch 1, dadurch gekennzeichnet, daß $R_1$ $CH_3$ ist, daß n = 1, und daß die Gruppe

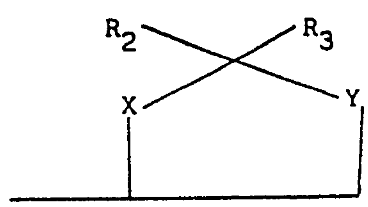

die Gruppe 1,4-Dioxa-spiro [4,5] decan-2-yl ist.

6. Verfahren nach Anspruch 1, dadurch gekennzeichnet, daß $R_1$ $CH_3$ ist, daß n=1, daß $R_2$ und $R_3$ jeweils $C_6H_5$ darstellen und X und Y jeweils O darstellen.

7. Verfahren nach Anspruch 1 zur Herstellung eines Aminocrotonats nach Formel III, dadurch gekennzeichnet, daß

1) ein Alkohol der Formel

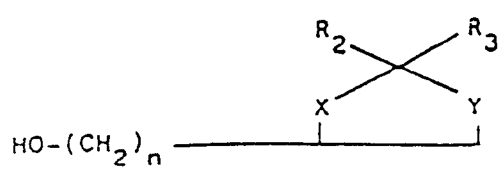

(IV)

worin n, X, Y, $R_2$ und $R_3$ die oben angegebene Bedeutung haben, umgesetzt wird mit 5-Acetyl-2,2-dimethyl-4,6-dioxo-1,3-dioxan der Formel

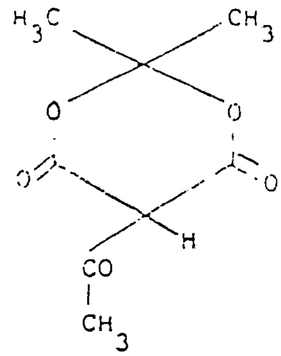

(V)

in einem aromatischen Lösungsmittel bei einer Temperatur, die zwischen der Raumtemperatur (15-20 °C) und der Refluxtemperatur des Reaktionsmediums liegt, während 1 bis 24 Std., um einem Ketoester der Formel

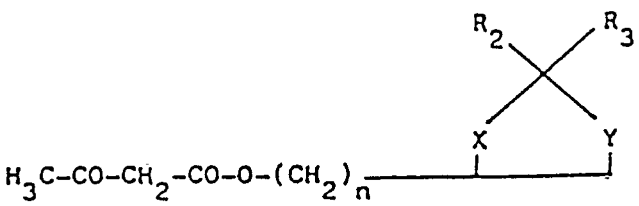

(VI)

zu erhalten,

2) das so erhaltene Derivat von Formel VI während 1 bis 20 Std. in einem chlorierten Lösungsmittel mit $NH_3$ umgesetzt wird.

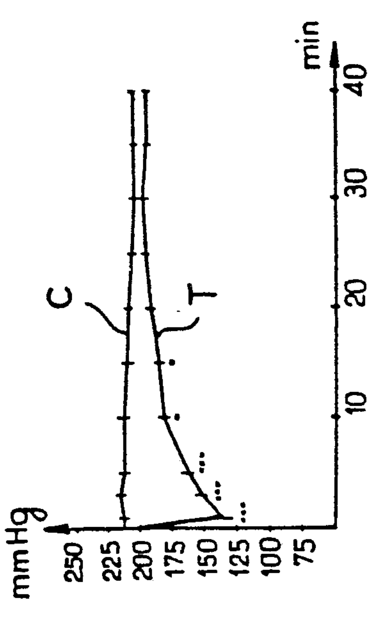


FIG.1

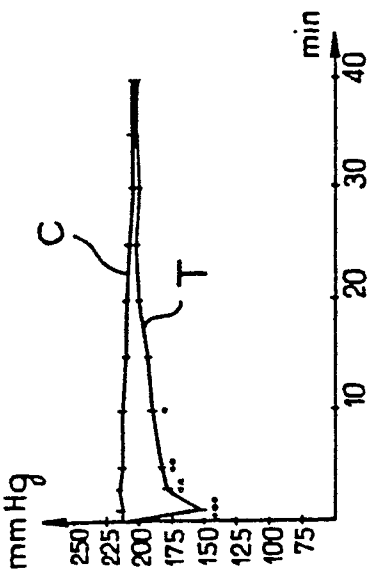


FIG.2

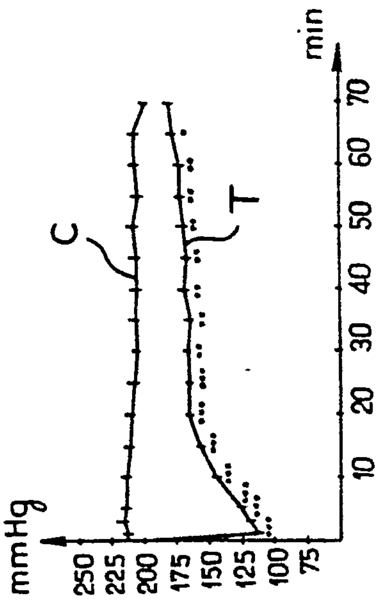


FIG.3

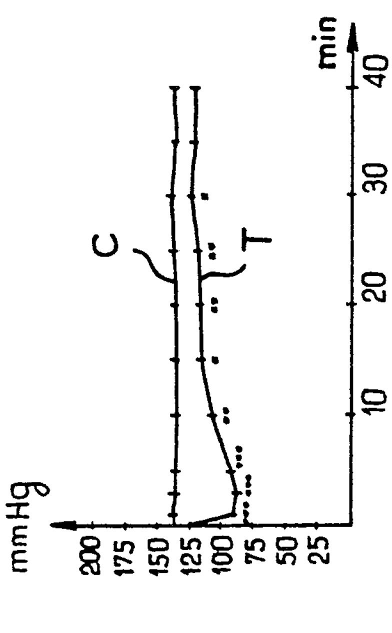


FIG.1a

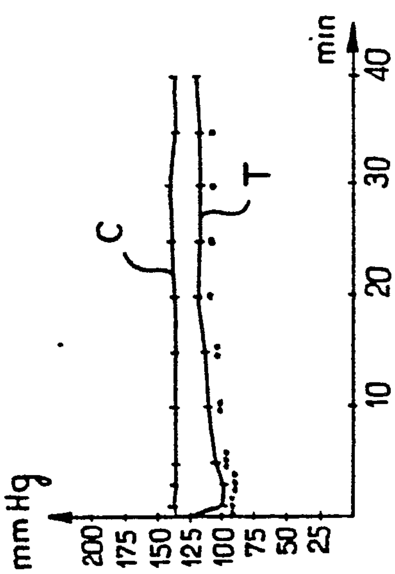


FIG.2a

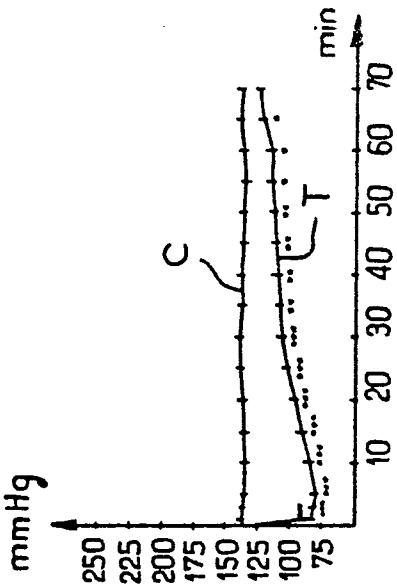


FIG.3a

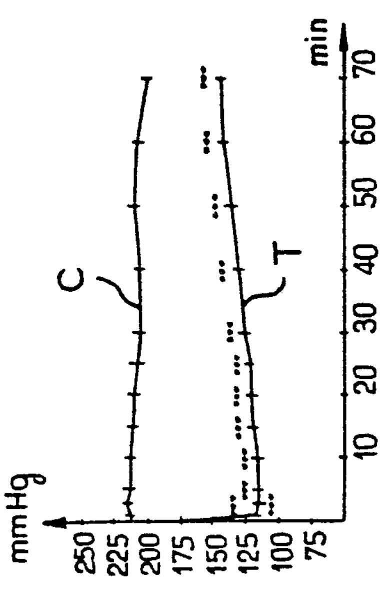


FIG.4

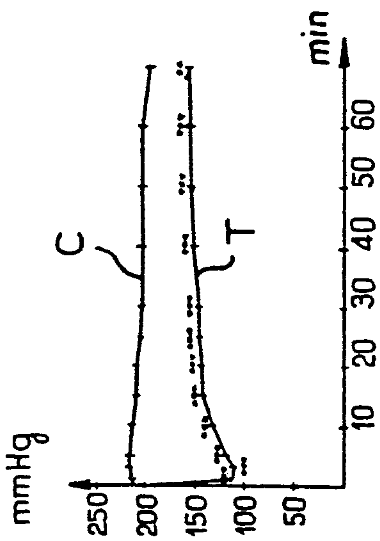


FIG.5

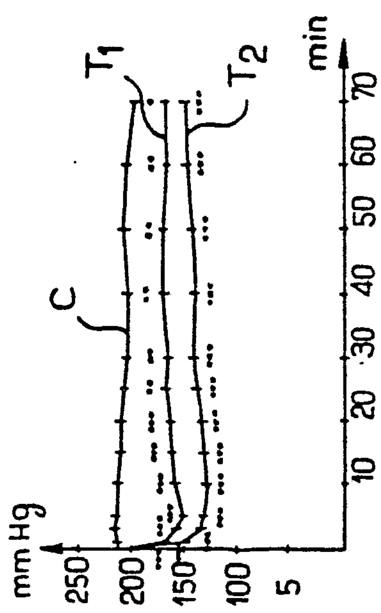


FIG.6

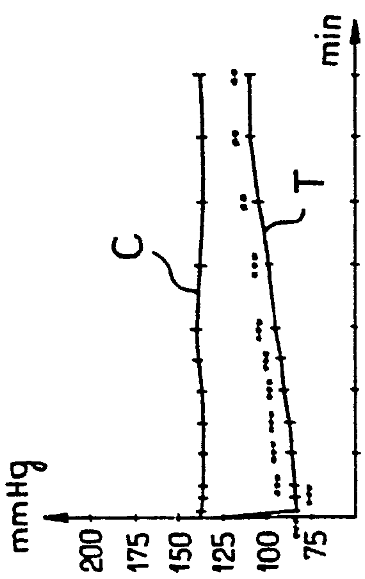


FIG.4a

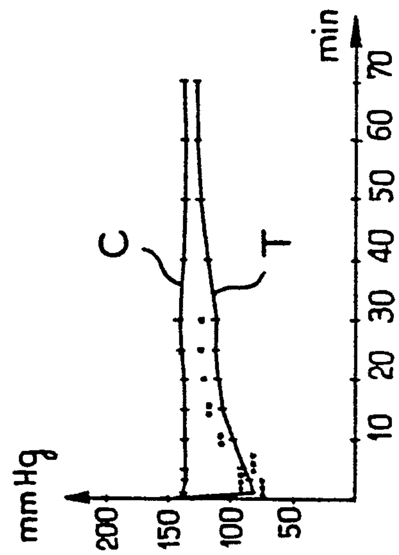


FIG.5a

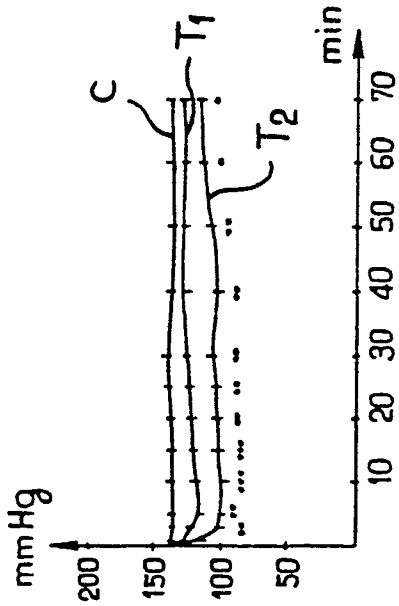


FIG.6a